# EUROPÄISCHE PATENTANMELDUNG

(11) **EP 2 140 857 A1**
(43) Veröffentlichungstag der Anmeldung: **06.01.2010**
(21) Anmeldenummer: 09161061.8
(22) Anmeldetag: 26.05.2009
(51) Int. Cl.: A61K 8/34, A61K 8/44, A61Q 19/00

(54) **Stabilisierung wässrig-alkoholischer Formeln durch Menthol**

(30) Priorität: 03.07.2008 DE 102008040105
(71) Anmelder: Beiersdorf AG, 20253 Hamburg (DE)
(72) Erfinder: Schmidt-Rose, Thomas, 20251 Hamburg (DE); Thiesen, Kathrin, 22303 Hamburg (DE); Köhler, Manuela, 20144 Hamburg (DE); Demitz, Michael, 22529 Hamburg (DE); Wöhrmann, Michael, 22525 Hamburg (DE); Frese, Christian, 22765 Hamburg (DE)

(57) **Zusammenfassung**

Verwendung von Menthol und/oder seinen Derivaten zur Stabilisierung kosmetischer Zubereitungen, die Kreatin und/oder seine Derivate, Ethanol und Wasser enthalten.

## Beschreibung

Die Erfindung betrifft die Verwendung von Menthol zur Stabilisation von wässrig alkoholischer Formeln mit einem Gehalt an Kreatin(derivaten).

Das Haar ist ein Hautanhangsgebilde und leitet sich von der obersten Hautschicht, der Epidermis ab. Wie diese besteht die Haarwurzel im Wesentlichen aus stark proliferierenden Epithelzellen, die final keratinisieren und dabei den äußerlich sichtbaren Haarschaft bilden.

Im untersten Abschnitt der Haarwurzel, dem sogenannten Bulbus, sitzen die Stammzellen die durch fortlaufende Zellteilung den Nachschub für die Haarschaftproduktion liefern. Diese Zellen gehören zu den proliferationsaktivsten Zellen des menschlichen Körpers.

Einzelne Haare wachsen bis zu 0,4 mm pro Tag, bezogen alle Haare der gesamten Kopfhaut sind dies aneinandergereiht rund 30-40m Keratinfasern. Dies ist eine enorme Syntheseleistung, die einen entsprechend hohen Energiebedarf zur Folge hat. Um die benötigte hohe Zufuhr von Nährstoffen und Sauerstoff sicherzustellen, besitzt jedes Haar ein spezielles Netzwerk von Blutgefäßen.

Haare wachsen nicht ein Leben lang kontinuierlich, sondern durchlaufen wiederholt einen Wachstumszyklus, der aus drei Phasen besteht. Die Anagenphase ist die eigentliche produktive Phase, gekennzeichnet durch intensive Zellteilung und Haarschaftbildung. Sie dauert für Kopfhaare zwischen 2 und 6 Jahren, wobei der Zeitraum für jedes Haar unterschiedlich ist (asynchrones Wachstum). Anschließend durchläuft die Haarwurzel eine Regressionsphase, das Katagen, in der sich der untere Teil der Wurzel weitgehend zurückbildet und die Zellvermehrung zum Stillstand kommt. In der sich anschließenden, etwa drei Monate dauernden Ruhephase (Telogen) findet keine Haarwachstum mehr statt, die Haarfaser steckt nur noch vorübergehend als Hornfaden in der Haut und fällt dann je nach mechanischer Belastung aus. Ein täglicher Verlust von rund 100 Haaren pro Kopf ist somit ein normaler Vorgang. Aus den zurückbleibenden hautoberflächennahen Teilen der Haarwurzel werden anschließend wieder Stammzellen mobilisiert, die den Bulbus regenerieren. Eine neue Anagenphase beginnt und ein neues Haar wächst an derselben Stelle.

Haarausfall kann zahlreiche Ursachen haben, meistens wird er jedoch, bei Männern wie bei Frauen, durch eine genetisch bedingte, erhöhte Sensitivität der Haarwurzelzellen gegenüber männlichen Geschlechtshormonen (Testosteron, Dihydrotestosteron) bedingt. Dies tritt bei Männern oft schon nach der Pubertät auf. Mehr als die Hälfte aller Männer zeigen im Alter von 40 Jahren die typischen Anzeichen dieser "androgenetischen Alopezie": Haarverlust im frontalen Bereich ("Geheimratsecken" und "hohe Stirn") sowie im okzipitalen Bereich.

Tatsächlich sind die Haare nicht komplett verschwunden, sondern sie sind von Haarzyklus zu Haarzyklus dünner und schwächer geworden und haben sich letztendlich in kleine unpigmentierte Haare, sogenannte Vellushaare, umgewandelt. Dabei hat sich auch die Zeitspanne der Wachstumsphase der betroffenen Haare verkürzt und das Verhältnis von wachsenden Anagenhaaren zu ruhenden Telogenhaaren hat sich zugunsten der ruhenden Haare verschoben.

Produkte die versprechen Haarausfall zu stoppen gibt es bereits seit langem auf dem Markt. Ein Beispiel ist hier das koffeinhaltige After Shampoo Liquid von Alpecin.

Andere gegen Haarausfall wirksame Wirkstoffe sind Kreatin und Carnitin.

Kreatin (von griechisch kreas = Fleisch) ist eine organische Säure, die in Wirbeltieren unter anderem zur Versorgung der Muskeln mit Energie beiträgt. Kreatin wird in der Niere, der Leber und in der Bauchspeicheldrüse synthetisiert. Sie leitet sich formal von den Aminosäuren Glycin und Arginin ab.

Kreatin spielt in Zellen eine wichtige Rolle im Energiestoffwechsel, es erlaubt größere Mengen an energiereichem Phosphat zu speichern, als es allein in Form des primären Energieträgers ATP möglich wäre.

Carnitin, genauer L-Carnitin, ist eine natürlich vorkommende, vitaminähnliche Substanz. Es spielt ebenfalls eine essentielle Rolle im Energiestoffwechsel menschlicher, tierischer und pflanzlicher Zellen. L-Carnitin fungiert als Transportmolekül für aktivierte Fettsäuren und erlaubt deren Transort aus dem Cytosol in die Mitochondiren, wo diese über die beta-Oxidation und den Citratzyklus abgebaut werden. Die dabei freiwerdende Energie wird zur Synthese von ATP genutzt, welches für Syntheseleistungen der Zellen benötigt wird. Langkettige Fettsäuren können nur als L-Carnitin-Ester durch die Mitochondrienmembranen transportiert werden.

Da die Haarwurzelzellen zu den proliferationsaktivsten Zellen des Körpers gehören und auf Grund ihrer ungeheuren Syntheseleistung einen hohen Energiebedarf besitzen, können Haarwurzeln durch den Einsatz von Kreatin und Carnitin stimuliert werden. Dadurch wird ihr Energiestoffwechsel unterstützt und somit kräftigeres, schneller wachsendes Haar erreicht. Außerdem ist eine Erhöhung des Anteils wachsender Haare gegenüber den in der Ruhephase befindlichen vorteilhaft.

Möchte man, dass die verwendeten Wirkstoffe auch wirklich wirken, so muss sichergestellt sein, dass diese auch bis zur Haarwurzel vordringen können. Eine einfache Möglichkeit liegt darin, die Konzentration der Wirkstoffe so hoch anzusetzen, dass genug die Kopfhaut und insbesondere die Wurzeln erreicht. Bei sehr gut wasserlöslichen Wirkstoffen ist dies kein Problem.

Verwendet man jedoch weniger gut wasserlösliche Substanzen wie etwa Kreatin und Carnitin, so stellt sich unter anderem das Problem ihrer geringen Löslichkeit. Setzt man höhere Konzentrationen ein, so kommt es bei der Lagerung der Produkte regelmäßig zum Auskristallisieren der Wirkstoffe. Dadurch sind diese dann nicht mehr bioverfügbar und können nicht wirksam sein. Darüber hinaus sind solche Produkte auch aus Verbrauchersicht unansehnlich und nicht akzeptabel.

Der Stand der Technik kennt in der DE 100 32964 kreatinhaltige kosmetische Emulsionen. In der WO 02076408 werden haarkosmetische Mittel mit einem Gehalt an Kreatin offenbart. Die WO 04/078117 offenbart haarwuchsfördernde Zubereitungen mit Kreatin und Menthol. Die WO 02/074265 beschreibt carnitinhaltige alkoholische Zubereitungen.

Es stellte sich auch die Aufgabe, die Kombination von Kreatin und Carnitin (0.3 - 2 % und 0.8 - 5%) als Wirkstoff in einer wässrigen oder wässrig-alkoholischen Lösung einzusetzen. Kreatin, welches nur mittelmäßig in Wasser löslich ist, erreicht hier leicht seine Löslichkeitsgrenze und es kann beim Lagern des Produktes zur Auskristallisation kommen. Diese Kristallisationsneigung wird häufig durch den Gehalt von weiteren üblichen kosmetischen Inhaltstoffen wie etwa Salze, Parfüm und vieles mehr verstärkt.

Menthol in Haarpflegeprodukten für Männer ist ebenfalls bereits bekannt. Menthol ist ein monocyclischer Monoterpen-Alkohol. Bei Raumtemperatur ist es ein farbloser, kristalliner Feststoff mit Pfefferminzgeruch.

Das Kristallsystem ist hexagonal, die Kristalle sind nadelförmig. Menthol kommt in vielen ätherischen Ölen, besonders in Pfefferminzölen vor. Das (-)-Menthol hat medizinische Anwendungen: So wird es zum Beispiel als Analgetikum bzw. schwaches Lokalanästhetikum verwendet. Außerdem findet Menthol als Duft- und Aromastoff Verwendung, sowie in der Bienenpflege gegen Milbenbefall.

Es wirkt am Kälte-Menthol-Rezeptor des Gehirns (TRPM8), daher hat Menthol einen (scheinbar) kühlenden Effekt beim Auftragen auf die Haut, die Körpertemperatur wird jedoch nicht beeinflusst. Diese Wirkung ist vergleichbar mit der von Capsaicin (scheinbar heißer Effekt).

Aus diesem Grund wird es auch in Kosmetikprodukten für Männer eingesetzt, da diese den kühlenden Effekt oft als erfrischend und angenehm beschreiben.

Es hat sich nun für den Fachmann unerwartet gezeigt, dass die Verwendung von Menthol und/oder seinen Derivaten Zubereitungen, die Kreatin und/oder seine Derivate, Ethanol und Wasser enthalten zu stabilisieren vermag. Dies betrifft insbesondere die Kombination von Kreatin und Carnitin (0.3 - 2 % und 0.8 - 5 %) in einer Wasser / Alkohol Mischung (7:3). Kreatin per se ist schlecht löslich. Nun hat sich überraschenderweise gezeigt, dass durch den Einsatz von 0.05 - 2 % Menthol und Mentholderivate wässrig ethanolische Produkte mit Kreatin sogar bei 6°C klar sind.

Bevorzugt ist es, wenn Menthol und/oder seine Derivate in Konzentrationen von 0,05 bis 2 Gew.-%, besonders bevorzugt 0,2 Gew.-% verwendet wird. Bevorzugt ist es, wenn Kreatin und/oder seine Derivate in Konzentrationen von 0,02 bis 2 Gew.-%, bevorzugt 0,4 bis 0,6 Gew.-%, besonders bevorzugt 0,2 Gew.-% vorliegen. Bevorzugt ist es, wenn Ethanol in einer Konzentration von wenigstens 20 Gew.-%, bevorzugt 25 Gew.-%, besonders bevorzugt 30 Gew.-%, ganz besonders bevorzugt 30 Gew.-% vorliegt. Bevorzugt ist es, wenn zusätzlich Carnitin enthalten ist, besonders bevorzugt in Konzentrationen von 0,8 bis 5 Gew.-%, weiter bevorzugt 0,9 bis 1,2 Gew.-%, besonders bevorzugt 1 Gew.-%. Bevorzugt ist es, wenn die Mentholderivate gewählt werden aus der Gruppe N-Ethyl-para-menthane-3-carboxamide (WS-3), Ethyl 3-(para-menthane-3-carboxamido)acetate (WS-5), (-)-Menthoxypropane-1,2,diol (Coolant Agent 10®, Takasago), Menthane-3,8-diol (Coolact 38D) und Menthol. Ganz besonders bevorzugt ist es, wenn Menthol verwendet wird. Bevorzugt ist es, wenn der Wassergehalt wenigstens 60 Gew.-%, bevorzugt 65 Gew.-%, besonders bevorzugt 66 Gew.-% beträgt. Bevorzugt ist es, wenn der Gehalt an Wasser und Ethanol zusammengenommen wenigstens 70 Gew.-%, bevorzugt 85 Gew.-%, besonders bevorzugt 95 Gew.-% beträgt. Bevorzugt ist es, wenn die Zubereitung gegen das Auskristallisieren von Kreatin durch einen Gehalt an Parfümöl stabilisiert wird.

### Beispiele:

Nachfolgend sind erfindungsgemäße und nichterfindungsgemäße Rezepturen angegeben.

Es wurde beobachtet, dass die Formel 1 direkt nach der Herstellung trüb ist und zum Aufrahmen des Kreatins neigt.

Auch Formel 2, welche zusätzlich Parfum enthält ist von Anfang an trübe und neigt zum Aufrahmen.

Formel 3 hingegen, welche zusätzlich Menthol enthält ist weder trübe noch rahmt sie innerhalb von 28 Tagen auf.

Überraschend wird beobachtet, das Menthol als Lösungsvermittler insbesondere für das Kreatin wirkt.

| inci | Handelsname | 1 | 2 | 3 | 4 | 5 | 6 | 7 |
|---|---|---|---|---|---|---|---|---|
| Aqua | | 67,13 | 66,73 | 66,53 | 66,78 | 66,78 | 66,78 | 66,78 |
| Citric Acid | | 0,006 | 0,006 | 0,006 | 0,006 | 0,006 | 0,006 | 0,006 |
| C12-15 Alkyl Benzoate | | 0,2 | 0,2 | 0,2 | 0,2 | 0,2 | 0,2 | 0,2 |
| Panthenol | | 0,15 | 0,15 | 0,15 | 0,15 | 0,15 | 0,15 | 0,15 |
| Aqua + Sodium Hydroxide | | 0,01 | 0,01 | 0,01 | 0,01 | 0,01 | 0,01 | 0,01 |
| PEG-40 Hydrogenated Castor Oil | | 0,3 | 0,3 | 0,3 | 0,3 | 0,3 | 0,3 | 0,3 |
| Menthol | | | | 0,2 | 0,2 | 0,1 | 0,3 | 0,4 |
| Alcohol Denat. | | 30 | 30 | 30 | 30 | 30 | 30 | 30 |
| Laureth-9 | | 0,5 | 0,5 | 0,5 | 0,5 | 0,5 | 0,5 | 0,5 |
| Carnitine | | 1,1 | 1,1 | 1,1 | 1 | 1 | 1 | 1 |
| Creatine | | 0,6 | 0,6 | 0,6 | 0,45 | 0,45 | 0,45 | 0,45 |
| Parfum | | | 0,4 | 0,4 | 0,4 | 0,4 | 0,4 | 0,4 |

### Anwendung:

Das Produkt wird in die Kopfhaut einmassiert und verbleibt dort bis zur nächsten Haarwäsche.

Die Stabilität wurde visuell an Raumtemperaturmustern nach 1, 7, 14, 28 Tagen Lagerung beurteilt.

## Patentansprüche

1. Verwendung von Menthol und/oder seinen Derivaten zur Stabilisierung kosmetischer Zubereitungen, die Kreatin und/oder seine Derivate, Ethanol und Wasser enthalten.

2. Verwendung nach Patentanspruch 1 **dadurch gekennzeichnet, dass** Menthol und/oder seine Derivate in Konzentrationen von 0,05 bis 2 Gew.-%, besonders bevorzugt 0,2 Gew.-% verwendet wird.

3. Verwendung nach einem der vorangehenden Patentansprüche **dadurch gekennzeichnet, dass** Kreatin und/oder seine Derivate in Konzentrationen von 0,02 bis 2 Gew.-%, bevorzugt 0,4 bis 0,6 Gew.-%, besonders bevorzugt 0,2 Gew.-% vorliegen.

4. Verwendung nach einem der vorangehenden Patentansprüche **dadurch gekennzeichnet, dass** Ethanol in einer Konzentration von wenigstens 20 Gew.-%, bevorzugt 25 Gew.-%, besonders bevorzugt 30 Gew.-%, außergewöhnlich bevorzugt 35 Gew.-% vorliegt.

5. Verwendung nach einem der vorangehenden Patentansprüche **dadurch gekennzeichnet, dass** zusätzlich Carnitin enthalten ist, besonders bevorzugt in Konzentrationen von 0,8 bis 5 Gew.-%, bevorzugt 0,9 bis 1,2 Gew.-%, besonders bevorzugt 1 Gew.-%.

6. Verwendung nach einem der vorangehenden Patentansprüche **dadurch gekennzeichnet, dass** die Mentholderivate gewählt werden aus der Gruppe N-Ethyl-para-menthane-3-carboxamide (WS-3), Ethyl 3-(para-menthane-3-carboxamido)acetate (WS-5), (-)-Menthoxypropane-1,2,diol (Coolant Agent 10®, Takasago), Menthane-3,8-diol (Coolact 38D) und Menthol, besonders bevorzugt **dadurch**, dass Menthol verwendet wird.

7. Verwendung nach einem der vorangehenden Patentansprüche **dadurch gekennzeichnet, dass** der Wassergehalt wenigstens 60 Gew.-%, bevorzugt 65 Gew.-%, besonders bevorzugt 66 Gew.-% beträgt.

8. Verwendung nach einem der vorangehenden Patentansprüche **dadurch gekennzeichnet, dass** der Gehalt an Wasser und Ethanol zusammengenommen wenigstens 70 Gew.-%, bevorzugt 85 Gew.-%, besonders bevorzugt 95 Gew.-% beträgt.

9. Verwendung nach einem der vorangehenden Patentansprüche **dadurch gekennzeichnet, dass** die Zubereitung gegen das Auskristallisieren von Kreatin durch einen Gehalt an Parfümöl stabilisiert wird.
